# EUROPEAN PATENT SPECIFICATION

(11) **EP 4 029 506 B1**
(45) Date of publication and mention of the grant of the patent: **16.04.2025**
(21) Application number: 20864172.0
(22) Date of filing: 09.09.2020
(51) Int. Cl.: A61K 9/08, A61K 31/69, A61K 41/00, A61K 47/12, A61K 47/26, A61P 35/00

(54) **METHOD FOR PREVENTING PRECIPITATION OF INJECTABLE SOLUTION CONTAINING P-BORONOPHENYLALANINE**
VERFAHREN ZUM VERHINDERN DER AUSFÄLLUNG EINER P-BORONOPHENYLALANIN ENTHALTENDEN INJIZIERBAREN LÖSUNG
PROCÉDÉ DE PRÉVENTION DE LA PRÉCIPITATION D'UNE SOLUTION INJECTABLE CONTENANT DE LA P-BORONOPHÉNYLALANINE

(30) Priority: 12.09.2019 JP 2019165969
(43) Date of publication of application: 20.07.2022
(73) Proprietor: Stella Pharma Corporation, Osaka-shi, Osaka 541-0043 (JP)
(72) Inventor: IGUCHI,Yoshiya, Osaka-shi, Osaka 5410043 (JP); KATAKUSE,Yoshimitsu, Osaka-shi, Osaka 5410043 (JP); NAKASHIMA,Hideki, Osaka-shi, Osaka 5410043 (JP)
(74) Representative: Cabinet Beau de Loménie
(86) International application number: PCT/JP2020/034085
(87) International publication number: WO 2021/049519

(56) References cited:
- WO-A2-2004/030661
- JP-A- 2009 051 766
- JP-A- 2013 173 804
- HALBERT, G. ET AL.: "Improved pharmaceutical stability of a boronphenylalanine mannitol formulation for boron neutron capture therap y", EUROPEAN JOURNAL OF PHARMACEUTICAL SCIENCES, vol. 48, no. 4-5, 2013, pages 735 - 739, XP029000008, ISSN: 0928-0987, DOI: 10.1016/j.ejps.2013.01.008

## Description

### BACKGROUND OF THE INVENTION

### TECHNICAL FIELD

The present invention relates to a method for preventing precipitation of an injection solution containing p-boronophenylalanine. More specifically, the present invention relates to a method for preventing precipitation of an injection solution containing p-boronophenylalanine under storage.

### BACKGROUND

Recently, attention has been drawn to a boron neutron capture therapy (BNCT) as a cancer treatment method utilizing a radioisotope. The boron neutron capture therapy is a treatment method in which a boron compound containing boron-10 isotope (¹⁰B) is delivered to cancer cells and the cancer cells are irradiated with a low energy neutron (for example, epithermal neutrons), and thus the cancer cells are locally destroyed by a nuclear reaction which arises in the cells. In this treatment method, since it is important to cause a boron compound which contains boron 10 to be selectively accumulated by cells of cancerous tissue so as to enhance therapeutic effect, it is necessary to develop boron compounds which are selectively and certainly taken by cancer cells.

Boron-containing compounds in which boron atoms or boron atomic groups are introduced into a basic structure have been synthesized as an agent used in BNCT. Examples of an agent used in the actual clinical practice include p-boronophenylalanine (BPA) and mercaptoundecahydrododecaborate (BSH).

p-Boronophenylalanine has very poor solubility at physiological pH.

In order to improve solubility of p-boronophenylalanine in water, a method of producing a fructose complex of BPA (for example, Patent Document 1), and a method of adding a monosaccharide or a polyol to p-boronophenylalanine in an alkaline solution (such as in an aqueous sodium hydroxide solution) and removing an inorganic salt with an ion exchange resin for use (for example, Patent Document 2) have been attempted.

Furthermore, another technique for improving solubility of p-boronophenylalanine has been proposed (Patent Document 3). Patent Document 4 discloses a liquid composition containing p-boronophenylalanine and sorbitol.

### Prior Art Document

### Patent Documents

Patent Document 1: US 5,492,900
Patent Document 2: US 6,169,076
Patent Document 3: JP-B-5345771
Patent Document 4: JP2009051766A

### SUMMARY OF THE INVENTION

Boron concentration in the blood at the time of administration required for exerting an effect as boron neutron capture therapy is limited. Therefore, it is desired to prepare a formulation having excellent stability while keeping a BPA concentration constant so as to maximally exhibit a therapeutic effect.

It was turned out, however, that, when the formulation is stored as an injection for a period until administration while keeping the BPA concentration constant, whereby sometimes a problem in stability occurs and precipitation occurs.

One of the objectives of the present invention is to provide a method for preventing precipitating an injection solution containing p-boronophenylalanine under storage in a wide temperature range, especially also including under low temperature storage.

The present inventors have intensively studied to solve the above problems and, as a result, have found that p-boronophenylalanine in an injection solution can be stabilized by the method of the claims in a wide temperature range, by incorporating a sugar alcohol and an antioxidant, and by changing a type of a pH adjusting agent according to a change in pH value, and thus the present invention has been completed.

That is, the present invention provides the following method.
[1] A method for preventing precipitation of an injection solution containing p-boronophenylalanine or a pharmaceutically acceptable salt thereof for boron neutron capture therapy comprising, preparing the injection solution which comprises p-boronophenylalanine or a pharmaceutically acceptable salt thereof, sorbitol, and a pH adjusting agent selected from citric acid or lactic acid, and pH of which is controlled to exceeding 7.5 and 8.0 or less.
[2] The method for preventing precipitation according to [1], wherein a concentration of the sugar alcohol is 2.6 to 6.5 w/v%.
[3] The method for preventing precipitation according to [1] or [2], wherein a content ratio of the sugar alcohol is in a range of 0.9 to 3.0, in molar ratio, with respect to a content of p-boronophenylalanine.
[4] The method for preventing precipitation according to any one of [1] to [3], wherein an amount of the pH adjusting agent selected from citric acid or lactic acid is set to 0 to 8.3 w/v% of the injection solution.
[5] The method for preventing precipitation according to any one of [1] to [4], wherein the injection solution is for an intravenous injection.

### [Injection Solution for Boron Neutron Capture Therapy] (p-Boronophenylalanine or pharmaceutically acceptable salt thereof)

The p-boronophenylalanine used in the present invention is not particularly limited, but has a ratio of boron 10 of boron atoms in a compound of preferably 75 % or more, more preferably 80 % or more, even more preferably 90 % or more, and particularly preferably 95 % or more.

In natural boron (boron), boron 10 and boron 11 are isotopes, and boron 10 is present in a ratio of 20 % and boron 11 in a ratio of 80 %. Therefore, prior to production of the injection solution containing p-boronophenylalanine of the present invention, boron having a mass number of 10 (boron 10) is concentrated. For this purpose, boron 10 and boron 11 in a natural boron compound are sorted out, and highly concentrated boron 10 is produced. As the boron used in the present invention, boron 10 may be concentrated to increase the concentration of boron 10, or a commercially available product may be used. As the commercially available product, for example, ¹⁰B concentrated boric acid (manufactured by Stella Chemifa Corporation) can be used as a starting material.

Here, as a method for measuring boron 10, it can be performed using Agilent 7500 (manufactured by Agilent), by a quadrupole ICP-MS (ICP-QMS) method using a quadrupole mass spectrometer part. ICP-QMS used for measurement is adjusted according to JIS K0133.

L-form is currently used as p-boronophenylalanine, and L-p-boronophenylalanine can be also preferably used in the present invention, but the present invention is not limited thereto. That is, racemic p-boronophenylalanine containing D-form or both D-form and L-form of p-boronophenylalanine can be used in the present invention.

Here, p-boronophenylalanine is, for example, synthesized by a known method (for example, H.R.Synder, A.J.Reedy, W.M.J.Lennarz, J.Am.Chem.Soc., 1958, 80, 835: C.Malan, C.Morin, SYNLETT, 1996, 167: US 5,157,149: JP-A-2000-212185: and JP-B-2979139), and can be used.

Here, the salt is not particularly limited as long as it is pharmacologically acceptable. Examples of the p-boronophenylalanine salt include salts with an organic acid, salts with an inorganic acid, salts with an organic base, and salts with an inorganic base.

Examples of the salts with an organic acid include acetates, trifluoroacetates, fumarates, maleates, lactates, tartrates, citrates, and methanesulfonates. Examples of the salts with an inorganic acid include hydrochlorides, sulfates, nitrates, hydrobromides, and phosphates. Examples of the salts with an organic base include salts with triethanolamine. Examples of the salts with an inorganic base include ammonium salts, sodium salts, potassium salts, calcium salts, and magnesium salts.

In the injection solution used in the present invention, a content of p-boronophenylalanine or a salt thereof based on a total amount of the injection solution is appropriately set depending on a balance with other components. The total content of p-boronophenylalanine and/or a salt thereof based on the total amount of the injection solution is not particularly limited, but is preferably 2.0 to 5.5 w/v%, more preferably 2.5 to 5.0 w/v%, and further preferably 2.5 to 4.0 w/v%.

When the content of p-boronophenylalanine in the injection solution of the present invention is within the above ranges, the amount of the injection solution falls within an appropriate liquid amount during clinical application, solution stability is good, and an effect during administration is excellent.

### (Sugar alcohol)

A sugar alcohol used in the present invention is sorbitol.

As sorbitol, D-sorbitol, which is currently approved for use in medicines and whose safety has been confirmed, can be preferably used, but is not limited thereto. That is, in the present invention, L-form or a mixture of L-form and D-form can be also used.

As mannitol, D-mannitol, which is currently approved for use in medicines and whose safety has been confirmed, can additionally be used, but is not limited thereto. That is, L-form or a mixture of L-form and D-form can be also used.

The total content of the sugar alcohol used in the injection solution of the present invention depends on the amounts of other additives, but is preferably 2.0 to 7.0 w/v%, more preferably 2.6 to 6.5 w/v%, and further preferably 2.6 to 4.2 w/v%, based on the total amount of the injection solution.

An amount of sugar alcohol is preferably in a range of 0.9 to 3.0, more preferably 0.9 to 2.0, and further preferably 1.1 to 1.5, in molar ratio, with respect to an amount of p-boronophenylalanine. When the amount of sugar alcohol is within these ranges, precipitation of p-boronophenylalanine can be suppressed and an osmotic pressure ratio can be adjusted appropriately.

### (Antioxidant)

An antioxidant can be optionally used in the injection solution used in the present invention. The antioxidant is not particularly limited as long as it is used as a component of an injection in the pharmaceutical field. The antioxidant is not limited, but is preferably one or more selected from a group consisting of sulfurous acid, bisulfite, pyrosulfurous acid, nitrous acid, ascorbic acid, L-cysteine, thioglycolic acid, and salts thereof.

Here, examples of the salts of sulfurous acid, bisulfite, pyrosulfurous acid, nitrous acid, ascorbic acid, L-cysteine or thioglycolic acid include alkali metal salts such as sodium salts and potassium salts; alkaline earth metal salts such as calcium salts and magnesium salts; and inorganic salts such as aluminum salts and ammonium salts. Furthermore, for example, a salt with an organic base such as trimethylamine, triethylamine, pyridine, picoline, ethanolamine, diethanolamine, triethanolamine, dicyclohexylamine or N,N'-dibenzylethylenediamine can also be used. Particularly preferred are the sodium salts, potassium salts, or ammonium salts.

Particularly preferred as the antioxidant used in the present invention is one or more selected from a group consisting of sodium sulfite, dried sodium sulfite, potassium sulfite, calcium sulfite, sodium bisulfite, potassium bisulfite, ammonium bisulfite, sodium pyrosulfite, and potassium pyrosulfite.

The total content of the antioxidant used in the injection solution of the present invention depends on the blending amounts of other additives, but is preferably 0.005 to 2.0 w/v%, more preferably 0.005 to 1.5 w/v%, further preferably 0.005 to 1.2 w/v%, even more preferably 0.01 to 0.6 w/v%, and most preferably 0.01 to 0.03 w/v%, based on the total amount of the injection solution.

### (Water)

The injection solution used in the present invention further contains water. A water used in the present invention is not particularly limited as long as it is used as a component of an injection in the pharmaceutical field.

A content of water used in the injection solution of the present invention depends on the blending amounts of other additives, but is preferably 80 w/v% or more and more preferably 85 w/v% or more, and preferably 95 w/v% or less and further preferably 94 w/v% or less, based on the total amount of the injection solution.

### (Osmotic pressure ratio)

An osmotic pressure ratio of the injection solution of the present invention is not particularly limited, but it is preferably within a range of 1.0 to 1.8 in comparison with physiological saline. More preferably, the osmotic pressure ratio is in a range of 1.1 to 1.5. Within these ranges, it becomes possible to reduce pain, avoid an onset of phlebitis, and shorten administration time in a case of a large amount of intravenous injection.

The injection solution used in the present invention may appropriately contain various metal ions that may be contained in vivo, in order to ensure stability in vivo and in vitro. Preferably, sodium ion is contained, and the concentration thereof is not particularly limited, but is particularly preferably from 130 mEq/L to 160 mEq/L. This numerical range which is close to a Na ion concentration range of a body fluid is preferable so that an electrolyte balance between an intracellular fluid and an extracellular fluid is not significantly disturbed.

### (pH Adjusting agent)

The injection solution used in the present invention can be appropriately added with a pH adjusting agent which is citric acid or lactic acid.

A content of the pH adjusting agent used in the injection solution used in the present invention depends on blending amounts of other additives, but, for example, as an inorganic acid such as hydrochloric acid, the content is preferably 0.001 to 0.5 w/v%, more preferably 0.001 to 0.10 w/v%, and further preferably 0.001 to 0.03 w/v%, based on the total amount of the injection solution.

The content of the pH adjusting agent used in the injection solution used in the present invention depends on the blending amounts of other additives, but, for example, as an organic acid such as citric acid, the content is preferably 0 to 8.3 w/v%, more preferably 0 to 1.7 w/v%, further preferably 0 to 0.56 w/v%, even more preferably 0 to 0.18 w/v%, and most preferably 0 to 0.08 w/v%, based on the total amount of the injection solution.

The content of the pH adjusting agent used in the injection solution used in the present invention depends on the blending amounts of other additives, but, especially when pH is in a range around 6.5 to 7.4 (not claimed) or 7.5, as an organic acid such as citric acid, the content is preferably 0 to 8.3 w/v%, more preferably 0.01 to 1.7 w/v%, further preferably 0.02 to 0.56 w/v%, even more preferably 0.03 to 0.18 w/v%, and most preferably 0.05 to 0.08 w/v%, based on the total amount of the injection solution.

As an inorganic alkaline component such as sodium hydroxide, the content is preferably 0 to 2.20 w/v%, more preferably 0.01 to 1.50 w/v%, further preferably 0.01 to 0.86 w/v%, and even more preferably 0.01 to 0.65 w/v%.

### (pH)

The pH of the injection solution used in the present invention is exceeding 7.5 to 8.0, and particularly from the viewpoint of preventing precipitation under storage at a region from room temperature to low temperatures, preferably in a range of pH exceeding 7.5 and 7.8 or less. A suitable pH adjusting agent, buffer and the like used in the art may be used to adjust the pH as needed. Claimed are citric acid and lactic acid as pH adjusting agents.

### [Other Components]

The injection solution used in the present invention may be added with a buffer such as a phosphate buffer solution, a tris-hydrochloric acid buffer solution, an acetate buffer solution, a carbonate buffer solution or a citrate buffer solution as needed. These buffers may be useful in stabilizing a preparation and reducing irritation. The claims demand the presence of citric acid or lactic acid as pH adjusting agents.

Further, the injection solution of the present invention can contain other components usually used in the technical field of the present invention as needed, unless contrary to the object of the present invention. Examples of such a component include additives usually used in a liquid, particularly an aqueous composition, for example, preservatives such as benzalkonium chloride, potassium sorbate and chlorohexidine hydrochloride, stabilizer such as edetic acid Na, thickening agents such as hydroxyethylcellulose and hydroxypropylmethylcellulose, isotonizing agents such as sodium chloride, potassium chloride, glycerin, sucrose and glucose, surfactants such as polysorbate 80 and polyoxyethylene hydrogenated castor oil, isotonic agents such as sodium chloride, potassium chloride and glycerin, and pH adjusting agents such as sodium hydroxide.

When the injection solution of the present invention is used as a medicine, it may be in a form of an injection for intravenous injection using a solution. In particular, it may be an intravenous drip infusion solution.

The injection solution is produced by dissolving, suspending or emulsifying a certain amount of an active ingredient in an aqueous solvent (for example, distilled water for injection, physiological saline, Ringer's solution, etc.), or an oil-based solvent (for example, vegetable oil such as olive oil, sesame oil, cottonseed oil or corn oil, propylene glycol, etc.) or the like, together with a dispersant (for example, polysorbate 80, polyoxyethylene hydrogenated castor oil 60, polyethylene glycol, carboxymethyl cellulose, sodium alginate, etc.), a preservative (for example, methylparaben, propylparaben, benzyl alcohol, chlorobutanol, phenol, etc.), an isotonizing agent (for example, sodium chloride, glycerin, D-mannitol, glucose, etc.) or the like. Additives such as a solubilizing agent (for example, sodium salicylate, sodium acetate, etc.), a stabilizer (for example, human serum albumin, etc.) and a soothing agent (for example, benzyl alcohol, etc.) may be used as desired. Further, an antioxidant, a colorant or the like and other additives may be added as needed.

In addition, a "pharmaceutically acceptable carrier" can also be used. Examples of such substances include solvents, solubilizing agents, suspending agents, isotonizing agents, surfactants, soothing agents and the like in liquid preparations. In addition, preparation additives such as preservatives (antiseptics) and colorants can be used according to a conventional method.

Preferable examples of the "solvent" include alcohols, propylene glycol, macrogol, and the like.

Examples of the solubilizing agent include polyethylene glycol, propylene glycol, benzyl benzoate, trisaminomethane, cholesterol, triethanolamine, sodium carbonate, sodium citrate, and the like.

Preferable examples of the "suspending agent" include hydrophilic polymers such as polyvinyl alcohol, polyvinylpyrrolidone, sodium carboxymethylcellulose, methylcellulose, hydroxymethylcellulose, hydroxyethylcellulose and hydroxypropylcellulose, and the like.

Preferable examples of the "isotonizing agent" include glucose, sodium chloride, glycerin, and the like.

Examples of the "surfactant" include sodium lauryl sulfate, lauryl aminopropionic acid, lecithin, benzalkonium chloride, benzethonium chloride, glyceryl monostearate, and the like.

Preferable examples of the "soothing agent" include benzyl alcohol and the like.

Preferable examples of the "preservative" include paraoxybenzoic acid esters, chlorobutanol, benzyl alcohol, phenethyl alcohol, dehydroacetic acid, sorbic acid, and the like.

### [Method for Producing Injection Solution] (not claimed)

A method for producing the injection solution includes mixing a pH adjusting agent such as sodium hydroxide, water and p-boronophenylalanine, and then adding a sugar alcohol. Here, in preparation, the order to put ingredients may be important for efficient production. Particularly preferably, a mixed solution of water and a pH adjusting agent of an alkaline component such as sodium hydroxide is first prepared, and then p-boronophenylalanine is added and stirred. Thereafter, a sugar alcohol is added and dissolved, a pH adjusting agent for an acidic component is added, and the volume is adjusted with water to prepare an injection solution. By following such a protocol, each component can be efficiently dissolved in a short time, and an excellent injection solution can be efficiently prepared.

Types and amounts of water, p-boronophenylalanine, sugar alcohol and pH adjusting agent are in accordance with the amounts described in the injection solution for boron neutron capture therapy.

### [Method for Preventing Precipitation of Injection Solution]

One of the methods for preventing precipitation of the injection solution of the present invention is a method for preventing precipitation of an injection solution for boron neutron capture therapy, in which the injection solution contains p-boronophenylalanine or a pharmaceutically acceptable salt thereof, the sugar alcohol,
and a pH adjusting agent, and the method includes controlling pH of the injection solution to exceeding 7.5 and 8.0 or less. Here, types and amounts of water, p-boronophenylalanine, sugar alcohol and pH adjusting agent are in accordance with the amounts described in the injection solution for BNCT.

Another aspect of the present invention is a method for preventing precipitation of an injection solution for boron neutron capture therapy, in which the injection solution contains p-boronophenylalanine or a pharmaceutically acceptable salt thereof, the sugar alcohol,
and a pH adjusting agent, the pH adjusting agent contains citric acid or lactic acid, and the method includes controlling pH of the injection solution to 7.5 to 8.0.
Types and amounts of water, p-boronophenylalanine, sugar alcohol and pH adjusting agent at this time are in accordance with the amounts described in the injection solution for boron neutron capture therapy.

Here, the term "preventing precipitation" refers to preventing precipitation when stored at various temperatures. That is, in particular, it includes preventing precipitation when stored at room temperature to low temperature suitable for storage, for example, 30°C or less, and preferably 25°C or less. For example, without limitation, it may be possible to prevent precipitation when stored at around 5°C. Here, the term "preventing precipitation" includes, for example, complete suppression of visual cloudiness, reduction of degree of cloudiness, extension of time until appearance of cloudiness, and the like. Also, the term "under storage" as used herein means to store at least 6 hours or more, preferably 24 hours or more, and more preferably 2 days or more. In some cases, it may be a long-term storage such as one week or one month.

### [Neutron Capture Therapy] (not claimed)

### (Administration)

As a use of the injection solution disclosed, utilization as an intravenous drip infusion is preferable, and an intravenous drip infusion to be used for boron neutron capture therapy is particularly preferable. Neutron capture therapy is a method of treating by a strong particle beam (alpha ray, 7Li particle) generated by a nuclear reaction between boron 10 taken into tumor cells and neutrons, and the injection solution can be used in this method with particular advantage.

Prior to irradiation, the injection solution can be previously administered to a subject or an animal, adjusted so as to collect boron 10 in the tumor, and then irradiated with epithermal neutron rays. Alternatively, prior to irradiation, the injection solution can be also previously administered to a subject or an animal, adjusted so as to collect boron 10 in the tumor, and then irradiated with epithermal neutron rays while further continuing administration. A dose of the injection solution is not particularly limited, but can be controlled to achieve a preferable intracellular boron concentration. Such a dose is set according to a type or progression of a tumor to be applied, age or weight of the subject and the like, but when the injection solution is used for intravenous administration, it is administered by an intravenous drip infusion at a rate of 200 to 500 ml per hour for 1.5 to 4.0 hours, and preferably for 2.0 to 3.6 hours. It is particularly preferable that the administration start timing be continuously from before the start of neutron irradiation to during the irradiation.

For example, without limitation, it is also effective that, to patients with brain tumors or patients with head and neck cancer, the injection solution is adjusted so that a BPA concentration is preferably 150 to 250 mg/kg/hour, and more preferably 200 mg/kg/hour, and administered for preferably 1.5 to 3 hours, and more preferably 2 hours, then deceleratingly administered so that the BPA concentration is preferably 80 to 120 mg/kg/hour, and more preferably 100 mg/kg/hour, and irradiated with epithermal neutron rays while performing the decelerating administration for a maximum of 0.5 to 1.5 hours, and preferably for a maximum of 1 hour.

Thus, the injection solution used is particularly preferably used for neutron capture therapy. A target disease is not limited, but solid cancer is preferable, and cancer originating from epithelial cells (epithelial tumor) can be particularly preferable. Typically, the target disease can be skin cancer including melanoma or the like, lung cancer, breast cancer, stomach cancer, colon cancer, uterine cancer, ovarian cancer, or head and neck cancer (oral cancer, laryngeal cancer, pharyngeal cancer, tongue cancer, etc.). Alternatively, even a sarcoma originating from non-epithelial cells can be targeted. Typically, a target sarcoma can be osteosarcoma, chondrosarcoma, rhabdomyosarcoma, leiomyosarcoma, fibrosarcoma, liposarcoma, and angiosarcoma. In addition to these, brain tumors such as glioma, primary central nervous system malignant lymphoma, meningioma, pituitary adenoma, schwannoma and craniopharyngioma can be target diseases for treatment. Not only initial and single cancer, but also cancer that has spread to individual organs, metastatic cancer, and intractable cancer can be targeted.

The present invention provides the following each embodiment of a method for preventing precipitation of an injection solution.
[1] A method for preventing precipitation of an injection solution containing p-boronophenylalanine or a pharmaceutically acceptable salt thereof for boron neutron capture therapy comprising,
   preparing the injection solution which comprises p-boronophenylalanine or a pharmaceutically acceptable salt thereof, sorbitol, agent selected from citric acid and lactic acid, and pH of which is controlled to exceeding 7.5 and 8.0 or less.
[2] The method for preventing precipitation according to any one of [1] wherein a concentration of the sorbitol is 2.6 to 6.5 w/v%.
[3] The method for preventing precipitation according to any one of [1] to [2] wherein a content ratio of the sorbitol is in a range of 0.9 to 3.0, in molar ratio, with respect to a content of p-boronophenylalanine.
[4] The method for preventing precipitation according to any one of [1] to [3], wherein the injection solution is for an intravenous injection.

### EXAMPLES

Hereinafter, the present invention will be described in more detail with reference to Examples, but these do not limit the scope of the present invention.

### (Production example)

Prior to production of an injection solution containing p-boronophenylalanine (BPA; L-form was used here) of the present invention, ¹⁰B concentrated boric acid, in which the content of ¹⁰B is 96 % (manufactured by Stella Chemifa Corporation) obtained by concentrating boron with a mass number of 10 (boron 10) was used. Using the highly concentrated boron 10 thus obtained, p-boronophenylalanine (BPA) was produced by a conventional method.

### [Reference Examples, Examples]

### (Preparation of BPA sorbitol aqueous solution; not according to the claims)

An aqueous solution containing 2.5 w/v% to 5.0 w/v% BPA and D-sorbitol, sodium bisulfite or sodium pyrosulfite was prepared as follows. That is, first, 5 g to 10 g of BPA was suspended in a solution prepared by dissolving 1.05 to 2.08 g of sodium hydroxide in 175 ml of water. 5.25 to 13.0 g of D-sorbitol was added thereto, and the mixture was stirred to dissolve the D-sorbitol. 0.02 g of sodium bisulfite or sodium pyrosulfite was added to the mixture and dissolved, and 1.22 ml (at pH 7.6) or an appropriate amount of 1 mol/l hydrochloric acid was added to adjust pH, and water was added to make a total amount of 200 ml. Then, the resulting solution was filtered with a 0.2 µm filter.

### (Preparation of aqueous BPA mannitol solution; not according to the claims)

Aqueous solutions were prepared in the same manner as the aqueous BPA sorbitol solution, using mannitol instead of sorbitol.

### (Preparation of aqueous BPA sugar alcohol solution)

Aqueous solutions were prepared in the same manner as the aqueous BPA sorbitol solution, allowing to coexist mannitol in addition to sorbitol.

### <Stability test 1>

Stability evaluation was carried out mainly using the following models and conditions as standard conditions for medicine severe stability test based on ICH guidelines.

First, as stability test 1, a storage test at 40°C was performed. In this storage test, the aqueous solutions were placed in storage device: LH21-13M (manufactured by NAGANO SCIENCE CO., LTD.), at 40°C ± 2°C, 75 ± 5% RH, in a dark place, for 2 weeks and 4 weeks, each solution was sampled, and BPA concentration, Tyr concentration, Phe concentration, and Ac-BPA concentration (high-performance liquid chromatograph Nexera X2 series, manufactured by Shimadzu Corporation) were measured and compared with those at the start of the test.

Here, measurement conditions by HPLC are as follows.
Column used: Mightysil RP-18GP (5 µm, 4.6 × 150 mm) manufactured by KANTO CHEMICAL CO., INC.
Mobile phase: 0.05 mol/L sodium dihydrogen phosphate reagent solution (pH 2.5)/methanol (95 : 5)
Column temperature: Constant temperature around 40°C
Flow rate: about 0.8 ml/min
Injection volume: 10 µl
Detection wavelength: 223 nm

Representative examples of results of stability evaluation 1 are shown in Tables 1 and 2. BPA residual amounts in the tables indicate residual amounts of BPA after 4 weeks from storage when the amount of BPA used for production in stability test 1 was 100%. Although not shown in the tables, an amount of initial tyrosine was evaluated as an index showing a state of initial BPA decomposition due to coexistence of components other than BPA in the composition.

**[Table 1]**

| | BPA Concentration | Additive 1 | Additive 2 | Measured osmotic pressure ratio | Measure d pH | BPA Residual amount after 4 weeks |
|---|---|---|---|---|---|---|
| Reference Example 1 | 2.5% | Sorbitol 2.625% | | 1.0 | 7.4 | |
| Example 1 | | | | 1.0 | 7.6 | |
| Example 2 | | | | 1.0 | 7.8 | |
| Reference Example 2 | 3.5% | Sorbitol 3.675% | Sodium pyrosulfit e 0.01% | 1.5 | 7.4 | 99% or more |
| Example 3 | | | | 1.4 | 7.6 | |
| Example 4 | | | | 1.4 | 7.8 | |
| Reference Example 3 | 4.0% | Sorbitol 4.2% | | 1.7 | 7.4 | |
| Example 5 | | | | 1.6 | 7.6 | |
| Example 6 | | | | 1.6 | 7.8 | |
| Reference Example 4 | 3.0% | Sorbitol 3.15% | | 1.2 | 7.4 | |
| Example 7 | | | | 1.2 | 7.6 | |
| Example 8 | | | | 1.2 | 7.8 | |
| Reference Example 5 | 3.0% | Sorbitol 4.7% | | 1.6 | 7.4 | |
| Example 9 | | | Sodium bisulfite 0.01% | 1.5 | 7.6 | 99% or more |
| Example 10 | | | | 1.5 | 7.8 | |
| Reference Example 6 | 3.0% | Sorbitol 5.75% | | 1.8 | 7.4 | |
| Example 11 | | | | 1.7 | 7.6 | |
| Example 12 | | | | 1.8 | 7.8 | |

| | | | | | | |
|---|---|---|---|---|---|---|
| (% of BPA and additives means w/v%) | | | | | | |

As shown in Table 1, the compositions of all the Examples showed good stability. Also, when the BPA concentration was set to 2.5 to 4.0 w/v% and sodium bisulfite was used as an antioxidant, the compositions similarly showed good stability. Furthermore, in cases where the BPA concentration was set to 2.5 w/v%, and the sorbitol concentration was increased to 5.35 w/v% or 6.5 w/v%, even when the type and concentration of the antioxidant were verified under the same conditions, compositions showing good stability were similarly obtained.

**[Table 2]**

| Examples | BPA Concentration | Additive 1 | Additive 2 | Measured osmotic pressure ratio | Measured pH | BPA Residual amount after 4 weeks |
|---|---|---|---|---|---|---|
| Reference Example 13 | 2.5% | Mannitol 2.625% | Sodium bisulfite 0.01% | 1.0 | 7.8 | 99% or more |
| Reference Example 7 | 2.5% | Mannitol 5.35% | Sodium bisulfite 0.01% | 1.6 | 7.4 | |
| Reference Example 14 | 2.5% | Mannitol 5.35% | Sodium bisulfite 0.01% | 1.6 | 7.6 | |
| Reference Example 15 | 2.5% | Mannitol 5.35% | Sodium bisulfite 0.01% | 1.6 | 7.8 | |

| | | | | | | |
|---|---|---|---|---|---|---|
| (% of BPA and additives means w/v%) | | | | | | |

In the storage test of the compositions of Table 2 as well, it was found that BPA was retained in the aqueous solutions of the Examples at 99% or more even after 4 weeks or more. In the retention property observation, no change in components were observed even from change in color and appearance.

By comprehensively determining the results of solubility and the storage test, it was found that the injection solutions containing sorbitol or mannitol of the Examples have excellent stability at a pH of 7.4 to 7.8, and 40°C storage, and also excellent homogeneity of the solution.

### [Examples, Comparative Examples]

### (Preparation of aqueous BPA sorbitol solution)

An aqueous solution containing 3 w/v% BPA, D-sorbitol and sodium bisulfite was prepared as follows. That is, first, 0.62 g of sodium hydroxide was added to 87 ml of water, and the mixture was stirred. 3 g of L-BPA was suspended therein. 3.15 g of D-sorbitol was added thereto, and the mixture was stirred to dissolve the D-sorbitol. 0.02 g of sodium bisulfite was added thereto, and an appropriate amount of 1 mol/l hydrochloric acid (not claimed) or 1 mol/l citric acid was added thereto at room temperature to adjust pH, and water was added to make a total amount of 100 ml.

### <Stability test 2>

The thus prepared aqueous BPA sorbitol solution was subjected to stability test 2. In this test, the aqueous BPA sorbitol solution was subjected to a storage test at 5°C. In this storage test, the sample was allowed to stand at 5°C ± 3°C/ambH/dark place, and the presence or absence of cloudiness and the time until cloudiness occurred were measured. The results are shown in Table 3.

**[Table 3]**

| | HCl | | Citric acid | | pH | Stirring time after pH adjustment | | Condition |
|---|---|---|---|---|---|---|---|---|
| Comparative Example 1 | 3.5 | ml (mmol) | 0 | ml (mmol) | 6. 8 | 40 | min | Cloudiness confirmed after stirring |
| Comparative Example 2 | 2.5 | ml (mmol) | 0 | ml (mmol) | 7.0 | 180 | min | Clearness confirmed after stirring → cloudiness confirmed after storage at 5°C for 7 days |
| Comparative Example 3 | 3.5 | ml (mmol) | 0 | ml (mmol) | 6.5 | 10 | min | Cloudiness confirmed after stirring |
| Example 16 | 0 | ml (mmol) | 0. 8 | ml (mmol) | 7.1 | | | Clearness confirmed after storage at 5°C for 7 days |
| Example 17 | 0 | ml (mmol) | 0. 8 | ml (mmol) | 7.2 | | | Clearness confirmed after storage at 5°C for 7 days |
| Example 18 | 0 | ml (mmol) | 0. 8 | ml (mmol) | 7.4 | | | Clearness confirmed after storage at 5°C for 7 days |

| | | | | | | | | |
|---|---|---|---|---|---|---|---|---|
| Comparative Example 1 : HCl 0.13w/v% Comparative Example 2 : HCl 0.09w/v% Comparative Example 3 : HCl 0.13w/v% Examples 16,17 and 18 : Citric acid 0.15w/v% | | | | | | | | |

As a result, it was found that in the low pH region, adjustment with only hydrochloric acid may cause cloudiness during storage at low temperature. On the other hand, cloudiness during storage at low temperature could be suppressed by adding citric acid.

Next, an aqueous solution containing 3 w/v% BPA, D-sorbitol, and sodium bisulfite was prepared as follows. That is, first, 0.32 g of sodium hydroxide was added to 43 ml of water, and the mixture was stirred. 1.50 g of L-BPA was suspended therein. 1.575 g of D-sorbitol was added thereto, and the mixture was stirred to dissolve the D-sorbitol. 0.01 g of sodium bisulfite was added thereto, and an appropriate amount of 1 mol/l hydrochloric acid (not claimed) or 1 mol/l citric acid was added thereto at room temperature to adjust pH, and water was added to make a total amount of 50 ml.

**[Table 4]**

| | Test Example 1 | Test Example 2 | Test Example 3 |
|---|---|---|---|
| pH | 6. 8 | 7.2 | 7.6 |
| Hydrochloric acid | 19 Hours | 66 Hours | No cloudiness up to 90 hours |

As a result, when hydrochloric acid was used, cloudiness might occur when stored at 5°C. Here, it was found that when citric acid was added instead of hydrochloric acid at a pH of 6.8, generation of cloudiness was delayed although there was cloudiness due to storage. As described above, it was found that it is possible to suppress cloudiness, such as completely preventing or delaying time of occurrence of cloudiness, by adding citric acid instead of hydrochloric acid.

## Claims

1. A method for preventing precipitation of an injection solution containing p-boronophenylalanine or a pharmaceutically acceptable salt thereof for boron neutron capture therapy comprising,
preparing the injection solution which comprises p-boronophenylalanine or a pharmaceutically acceptable salt thereof, sorbitol, and a pH adjusting agent selected from citric acid or lactic acid, and pH of which is controlled to exceeding 7.5 and 8.0 or less.

2. The method for preventing precipitation according to claim 1, wherein a concentration of the sorbitol is 2.6 to 6.5 w/v%.

3. The method for preventing precipitation according to claim 1 or 2, wherein a content ratio of the sorbitol is in a range of 0.9 to 3.0, in molar ratio, with respect to a content of p-boronophenylalanine.

4. The method for preventing precipitation according to any one of claims 1 to 3, wherein an amount of the pH adjusting agent selected from citric acid or lactic acid is set to 0 to 8.3 w/v% of the injection solution.

5. The method for preventing precipitation according to any one of claims 1 to 4, wherein the injection solution is for an intravenous injection.

## Patentansprüche

1. Verfahren zum Verhindern einer Ausfällung einer Injektionslösung, die p-Boronophenylalanin oder ein pharmazeutisch unbedenkliches Salz davon enthält, in der Bor-Neutroneneinfangtherapie, das umfasst:
Zubereiten der Injektionslösung, die p-Boronophenylalanin oder ein pharmazeutisch unbedenkliches Salz davon, Sorbit und ein pH-Einstellmittel umfasst, das aus Zitronensäure oder Milchsäure ausgewählt ist, und mit einem pH-Wert, der auf mehr als 7,5 und 8,0 oder weniger gesteuert wird.

2. Verfahren zum Verhindern einer Ausfällung nach Anspruch 1, wobei eine Konzentration des Sorbits 2,6 bis 6,5 Gew./Vol.-% beträgt.

3. Verfahren zum Verhindern einer Ausfällung nach Anspruch 1 oder 2, wobei ein Gehaltsmolverhältnis des Sorbits in Bezug auf einen Gehalt von p-Boronophenylalanin in einem Bereich von 0,9 bis 3,0 liegt.

4. Verfahren zum Verhindern einer Ausfällung nach einem der Ansprüche 1 bis 3, wobei eine Menge des pH-Einstellmittels, das aus Zitronensäure oder Milchsäure ausgewählt ist, auf 0 bis 8,3 Gew./Vol.-% in Bezug auf die Injektionslösung festgelegt wird.

5. Verfahren zum Verhindern einer Ausfällung nach einem der Ansprüche 1 bis 4, wobei die Injektionslösung für eine intravenöse Injektion vorgesehen ist.

## Revendications

1. Procédé pour empêcher la précipitation d'une solution injectable contenant de la p-boronophénylalanine ou un sel pharmaceutiquement acceptable de celle-ci pour une thérapie de capture de neutrons par le bore comprenant
la préparation de la solution injectable qui comprend de la p-boronophénylalanine ou un sel pharmaceutiquement acceptable de celle-ci, du sorbitol, et un agent d'ajustement du pH choisi parmi l'acide citrique et l'acide lactique, et dont le pH est régulé pour dépasser 7,5 et être de 8,0 ou moins.

2. Procédé pour empêcher la précipitation selon la revendication 1, dans lequel la concentration du sorbitol est de 2,6 à 6,5 % en poids/volume.

3. Procédé pour empêcher la précipitation selon la revendication 1 ou 2, dans lequel le rapport molaire de la teneur en sorbitol par rapport à la teneur en p-boronophénylalanine est situé dans la plage allant de 0,9 à 3,0.

4. Procédé pour empêcher la précipitation selon l'une quelconque des revendications 1 à 3, dans lequel la quantité de l'agent d'ajustement du pH choisi parmi l'acide citrique et l'acide lactique est établie à 0 à 8,3 % en poids/volume de la solution injectable.

5. Procédé pour empêcher la précipitation selon l'une quelconque des revendications 1 à 4, dans lequel la solution injectable est destinée à une injection intraveineuse.
